# EUROPÄISCHE PATENTSCHRIFT

(11) **EP 1 968 722 B1**
(45) Veröffentlichungstag und Bekanntmachung des Hinweises auf die Patenterteilung: **15.02.2012**
(21) Anmeldenummer: 06829663.1
(22) Anmeldetag: 15.12.2006
(51) Int. Cl.: B01D 15/36, B01D 15/08, C07K 1/16, B01J 20/283, B01J 20/286

(54) **VERFAHREN ZUR ABTRENNUNG VON PROTEINEN AUS FLÜSSIGEN MEDIEN**
METHOD FOR SEPARATING PROTEINS FROM LIQUID MEDIA
PROCEDE DE SEPARATION DE PROTEINES A PARTIR DE MILIEUX LIQUIDES

(30) Priorität: 16.12.2005 DE 102005060392
(43) Veröffentlichungstag der Anmeldung: 17.09.2008
(73) Patentinhaber: SÜD-CHEMIE AG, 80333 München (DE)
(72) Erfinder: SOHLING, Ulrich, 85356 Freising (DE); SCHEPER, Thomas, 30559 Hannover (DE); KASPER, Cornelia, 30159 Hannover (DE); RIECHERS, Daniel, 30451 Hannover (DE); BURZLAFF, Arne, 30451 Hannover (DE)
(74) Vertreter: Stolmár, Matthias
(86) Internationale Anmeldenummer: PCT/EP2006/012130
(87) Internationale Veröffentlichungsnummer: WO 2007/073893

(56) Entgegenhaltungen:
- EP-A2- 0 180 934
- WO-A-93/15832
- WO-A-2004/103552
- DE-A1-102005 012 369
- JP-A- 1 284 310
- KR-A- 2001 085 045

## Beschreibung

Die Erfindung betrifft ein Verfahren zur Abtrennung von Proteinen aus flüssigen Medien.

Die Produktion biologisch aktiver Substanzen gelingt heute mit Hilfe der Zellkulturtechnik in großem Maßstab. Die etablierten Verfahren ermöglichen sowohl die Produktion von Enzymen für die technische Anwendung, wie z.B. die Nahrungsmittelherstellung, als auch die Produktion rekombinanter Proteine mit pharmazeutischer Bedeutung. Dafür werden die Zielproteine von Mikroorganismen oder Säugetierzellen produziert und anschließend aus dem Kultivierungsmedium oder den Zellen isoliert. Dieser Herstellungsprozess gliedert sich in die Fermentation mit vorgelagerter Medienaufbereitung und in eine nachfolgende Prozesskette, welche zur Reindarstellung des Zielproteins dient. Die Kosten der Prozessschritte zur Proteinaufreinigung übertreffen häufig 80% der Gesamtherstellungskosten. Zur Aufreinigung werden meist chromatographische Methoden verwendet, um einerseits das Zielprotein im Prozessstrom anzureichern und um andererseits Fremdproteine und anderer Kontaminationen, wie Endotoxine, Viren oder DNA, abzutrennen. Werden die Chromatographiematerialien mehrfach verwendet, müssen sie fortwährend auf ihre Trennleistung oder eine eventuelle mikrobielle Kontamination überprüft werden. Durch Verwendung von Einmal-Materialien können die Kosten für Validierung und Reinigung teilweise eingespart werden. Für einen erfolgreichen Einsatz muss ein solches Einmal-Chromatographiematerial eine reproduzierbar hohe Trennleistung erbringen und einen niedrigen Investitionsaufwand pro Verbrauchseinheit erfordern. Es sollte hohe Bindungskapazitäten und eine schnelle Bindungskinetik für die interessierenden Zielproteine oder die störenden Kontaminationen aufweisen.

Bentonite und insbesondere deren mineralische Hauptkomponente Montmorillonit eignen sich zur reversiblen Bindung von Proteinen. Bentonite und andere vergleichbare Schichtsilicate wirken dabei als natürliche Kationenaustauscher und können Proteine bei pH-Werten unterhalb ihres isoelektrischen Punktes adsorbieren. Natriumbentonit besitzt jedoch die Eigenschaft, in Wasser stark zu quellen. Werden wenige Gramm Natriumbentonit in eine wässrige Lösung eingebracht, führt dies zu einem starken Anstieg der Viskosität, was die Handhabung der flüssigen Medien stark erschwert. Ferner lässt sich Natriumbentonit nur mit hohem Aufwand als Filtermedium oder als Packung in einer Chromatographiesäule verwenden, da durch die starke Quellung das Volumen der Filterpackung bzw. der Säulenpackung stark zunimmt und der Filter bzw. die Säule dadurch verstopft wird. Für eine technische Anwendung besteht daher bisher nur die Möglichkeit, den Natriumbentonit jeweils batchweise zum zu reinigenden flüssigen Medium zu geben und nach der Adsorption der Proteine den Bentonit wieder abzufiltrieren bzw. das gereinigte flüssige Medium durch Abpressen vom Bentonit abzutrennen.

Über die Verwendung von Schichtsilicaten zur Abtrennung von Enzymen aus dem filtrierten Saft homogenisierter Zuckerrüben berichten C. Buttersack, K. Novikow, A. Schaper und K. Buchholz (Zuckerind. 119 (1994), Nr. 4, S. 284-291). Durch Adsorption an Natriumbentonit und anschließende Desorption mit einem pH-Puffer konnten auf einfache Weise Enzyme in reiner Form abgetrennt werden. Die Arbeiten zeigen die gute Eignung von Schichtmineralien zur Abtrennung bzw. Anreicherung von Proteinen. Die Handhabung der Schichtmineralien ist jedoch kompliziert, da diese in Wasser zu einer starken Erhöhung der Viskosität der Aufschlämmung führen und die Schichtmineralien in sehr kleine Partikel zerfallen. Es ist daher sehr schwierig, die Anreicherung von Proteinen in der Weise durchzuführen, dass das Schichtmaterial in eine Säule gepackt wird, über welche dann die das Protein enthaltende Flüssigkeit geleitet wird. Ohne Hilfsmaßnahmen verstopft die Säule relativ rasch, da das Schichtmineral stark quillt. Um dennoch eine Abtrennung der Enzyme in einer Säule zu ermöglichen, schlagen die Autoren vor, den Natriumbentonit in ein Calciumalginat-Gel einzugießen. Das hydrierte Homogenat wird bei pH = 5,0 über die Säule gepumpt, die mit Perlen des immobilisierten Bentonits gefüllt ist. Nachdem die nicht adsorbierte Fraktion aus der Säule ausgetreten ist, wird mit einer auf pH = 8 eingestellten Pufferlösung das Enzym eluiert. Für die großtechnische Abtrennung von Proteinen ist die Verwendung von Natriumbentonit, welcher wie von den Autoren beschrieben in einem Calciumalginat fixiert ist, wegen der hohen anfallenden Kosten weniger geeignet.

In der DE 1 160 812 A1 wird ein Verfahren zur Erhöhung der Eiweißstabilität von Bier unter Verwendung eines weitporigen Kieselgels als Adsorptionsmittel beschrieben. Dabei wird das Bier mit einem feingemahlenen Kieselgel behandelt, das eine Oberfläche von 200 bis 400 m²/g, ein Porenvolumen von mehr als 0,6 ml/g und einen Porendurchmesser von mehr als 60 Å besitzt.

In der GB 752,669 wird ein Verfahren zur Abtrennung siliziumhaltiger Verunreinigungen aus Lösungen beschrieben, die Insulin enthalten. Bei der Herstellung von Insulin kann dieses aus organischen Lösungen abgetrennt werden, indem es auf Siliziumdioxid oder einem Ton adsorbiert wird, sodass das Lösungsmittel abgetrennt werden kann. Anschließend kann das Insulin mit einer stark alkalischen wässrigen Lösung wieder eluiert werden. Durch den stark alkalischen pH-Wert wird die wässrige Insulinlösung jedoch durch siliziumhaltige Verunreinigungen kontaminiert, die sich vom Adsorptionsmittel ablösen. Diese Verunreinigungen liegen in kolloidaler Form vor, sodass ihre Abtrennung durch Filtration schwierig ist. Zur Abtrennung dieser Verunreinigungen wird vorgeschlagen, die wässrige Lösung auf einen pH-Wert von 6,5 bis 9,0 einzustellen und anschließend stehen zu lassen. Es bildet sich ein Niederschlag, der leicht durch Filtration abgetrennt werden kann.

In der US 4,605,621 wird ein Verfahren zur Immobilisierung von Enzymen beschrieben, wobei das Enzym mit einem organisch modifizierten Ton umgesetzt wird. Als Ton wird ein Smektit, Hectorit oder Vermiculit verwendet, bei dem zumindest ein Teil der Kationen durch Oniumionen und/oder organometallische Kationen ersetzt wurde, sodass der Ton hydrophobe Eigenschaften erhält.

In der US 4,126,605 wird ein Verfahren zur Herstellung von γ-Globulinen beschrieben, wobei Fraktionen von γ-Globulinen, die aus Blutplasma gewonnen wurden, weiter gereinigt werden. Dazu wird das γ-Globulin zunächst in einer wässrigen Lösung aufgenommen, die ein Hydrocolloid, wie Hydroxyethylstärke, Gelatine, Dextrose oder Albumin enthält und durch einen Puffer auf einen pH-Wert im Bereich von 3,5 bis 8,0 eingestellt ist. Zur weiteren Reinigung kann diese Lösung mit einer Aufschlämmung eines Tonminerals vermischt werden. Als Tonmineralien können beispielsweise Bentonit oder Vermiculit verwendet werden. Durch Zugabe eines organischen Lösungsmittels, beispielsweise eines Polyethylenglykols, in einer Menge von etwa 10 Gew.-% wird eine erste Fraktion ausgefällt, die im Wesentlichen Verunreinigungen enthält. Diese kann beispielsweise durch Zentrifugieren abgetrennt werden. Bei einem pH-Wert von etwa 7,0 bis 7,2 wird dann weiteres organisches Lösungsmittel bis zu einem Anteil von 16 bis 24 Gew.-% zugegeben. Dabei fällt eine sehr reine Fraktion von γ-Globulinen aus, die dann beispielsweise durch Zentrifugieren abgetrennt werden kann.

In der EP 0 071 647 A1 wird ein Verfahren zur Abtrennung von Interferonen aus wässrigen Lösungen beschrieben. Dazu wird die Lösung mit einer Zusammensetzung vermischt, die Kieselsäure enthält. Als eine solche Zusammensetzung kann beispielsweise Bentonit, ein saurer Ton, Kaolin oder ein Magnesiumaluminiumsilikat verwendet werden. Das Interferon wird von der Zusammensetzung adsorbiert und kann mit einer wässrigen Lösung die eine nicht ionische oberflächenaktive Substanz enthält, eluiert werden. Geeignete oberflächenaktive Verbindungen sind beispielsweise Sorbitanmonoalkylester, Polyalkylether oder Polyoxyethylen-Polyoxypropylen-Copolymere.

In der KR 2001 085 045 wird ein Verfahren beschrieben, mit welchem sich Proteine aufkonzentrieren und reinigen lassen. Dazu werden die Proteine, welche in einem Puffer gelöst sind, der einen pH von 4 bis 8 aufweist, an Materialien wie Kaolinit, Siliziumdioxid oder Silikate gebunden. Mit einer Reinigungslösung werden nicht gebundene Proteine entfernt. Die Proteine werden mit einem Elutionsmittel aus der festen Phase abgetrennt. Die Elutionslösung weist einen pH-Wert im Bereich von 4 bis 8 auf und enthält 5 bis 30 % Alkohol, bevorzugt niedere Alkohole, wie Methanol, Ethanol und Isopropanol. Das Eluat weist einen pH-Wert von 7 bis 10 auf und enthält 0,5 bis 2 % SDS. Die als feste Phase verwendeten Materialien Kaolinit, Siliziumdioxid und Silikate weisen eine Partikelgröße im Bereich von 0,1 bis 10 Mikrometer auf.

In der EP 0 180 934 A2 wird ein Verfahren zur Herstellung grob gekörnter Schichtsilikate beschrieben. Dazu wird eine Suspension eines Schichtsilikats in einer wässrigen Polymerlösung hergestellt und die erhaltene Polymerlösung in eine Elektrolytlösung eingetropft, sodass eine vernetzung des Polymers eintritt. Die entstandenen Körner oder Kugeln werden dann abgetrennt. Die grob gekörnten Schichtsilikate, die in einer Polymermatrix eingeschlossen sind, können als Adsorbentien für Proteine verwendet werden.

In der WO 93/15832 wird ein Adsorptionsmittel und dessen Verwendung zur Klärung wässriger Flüssigkeiten beschrieben, insbesondere von Getränken, wie Bier, Wein und Fruchtsäften. Das Adsorptionsmittel wird hergestellt, indem ein natürlicher Ton mit heißer Mineralsäure extrahiert wird, bis der SiO₂-Gehalt mindestens 80 Gew.-%, vorzugsweise mindesten 90 Gew.-%, insbesondere bevorzugt zumindest 95 Gew.-% beträgt, und das Material röntgenamorph ist. Der Gehalt an nicht extrahierbarem Aluminium beträgt bevorzugt höchsten 1 Gew.-%, besonders bevorzugt höchstens 2 Gew.-%, berechnet als Al₂O₃.

In der nachveröffentlichten DE 10 2005 012 639 A1 wird ein Verfahren zur Abtrennung von Biomolekülen aus flüssigen Medien beschrieben, wobei als Adsorptionsmittel ein Tonmaterial eingesetzt wird. Das Verfahren dient zur Abtrennung störender Proteine aus Flüssigkeiten.

In jüngerer Zeit ist eine neue Klasse von Tonmaterialien aufgefunden worden, die sehr gute Eigenschaften bei der Adsorption von Biomolekülen aufweisen. Diese Materialien sind wie die bisher verwendeten Tone Verwitterungsprodukte. Sie zeigen jedoch keine kristallinen Eigenschaften mehr und sind daher röntgenamorph. Sie besitzen einen im Vergleich zu den bisher verwendeten Tonen sehr hohen Anteil an SiO₂ und ein sehr hohes Porenvolumen. Im Gegensatz zu beispielsweise Natriumbentoniten quellen sie in Wasser nicht. In der DE 10 2005 012 639 A1, welche nach dem Zeitrang dieser Anmeldung veröffentlicht wurde, wird ein Verfahren beschrieben, bei welchem ein solches Tonmaterial für die Abtrennung störender Biomoleküle aus flüssigen Medien eingesetzt wird. So ist das Tonmaterial beispielsweise zum Klären von Wein oder zum Stabilisieren von Bier geeignet.

Der Erfindung lag die Aufgabe zugrunde, ein Verfahren bereitzustellen, mit welchem auch in großtechnischem Maßstab eine Abtrennung von Proteinen aus flüssigen Medien gelingt, wobei in einer bevorzugten Ausführungsform es auch möglich sein soll, die abgetrennten Proteine zurückzugewinnen, ohne dass diese einen übermäßigen Aktivitätsverlust erleiden.

Die Aufgabe wird mit einem Verfahren mit den Merkmalen des Patentanspruchs 1 gelöst. Vorteilhafte Weiterbildungen des erfindungsgemäßen Verfahrens sind Gegenstand der abhängigen Ansprüche.

Bei dem erfindungsgemäßen Verfahren wird zur Abtrennung von Proteinen aus flüssigen Medien:
- ein Proteine enthaltend flüssiges Medium bereitgestellt;
- ein Tonmaterial bereitgestellt, welches:
   - eine spezifische Oberfläche von mehr als 150 m²/g,
   - ein Porenvolumen von mehr als 0,35 ml/g,
   - eine Ionenaustauschkapazität von mehr als 40 meq/100 g, und
   - ein Sedimentvolumen in Wasser von weniger als 15 ml/2g, und
   - bezogen auf das wasserfreie Tonmaterial (atro), einen Al₂O₃-Gehalt im Bereich von 4 bis 11 Gew.-% aufweist;
- das Tonmaterial auf einen pH-Wert von 3,5 bis 9,0 äquilibriert wird,
- das flüssige Medium mit dem äquilibrierten Tonmaterial behandelt wird, und
- das gereinigte, an Proteinen abgereicherte flüssige Medium von dem Tonmaterial abgetrennt.

Überraschenderweise wurde gefunden, dass ein Tonmaterial, welches die oben beschriebenen Eigenschaften aufweist, Proteine in Mengen binden kann, welche für eine technische Anwendung interessant sind. Durch die Äquilibrierung des Tonmaterials auf einen pH-Wert von 3,5 bis 9,0 wird eine irreversible Deaktivierung bzw. Denaturierung der am Tonmaterial adsorbierten Proteine verhindert, sodass diese ggf. wiedergewonnen werden können, ohne dass ein starker Verlust ihrer Aktivität in Kauf genommen werden muss. Die Äquilibrierung wird bevorzugt in der Weise durchgeführt, dass das Tonmaterial mit einem Puffer behandelt wird, der einen pH-Wert aufweist, bei dem auch das interessierende Protein stabil ist bzw. keine übermäßige Deaktivierung erleidet. Bevorzugt wird für die Äquilibrierung ein Puffer verwendet, der anschließend auch für die Reinigung des flüssigen Mediums bzw. des interessierenden Proteins verwendet wird. Dieser Puffer ist vorzugsweise auf etwa den gleichen pH-Wert eingestellt, wie er auch beim flüssigen Medium eingestellt ist. Die Äquilibrierungsdauer hängt beispielsweise von der Menge des Tonmaterials ab, oder, falls das Tonmaterial in Form einer Packung oder Säule bereitgestellt wird, von deren Dimensionierung. Bei sehr kleinen Säulen, kann die Dauer der Äquilibrierung ggf. nur wenige Sekunden betragen während bei sehr großen Säulen oder Filterpackungen auch längere Zeiträume erforderlich sein können. Vorzugsweise wird die Äquilibrierung für eine Dauer von zumindest 10 Sekunden, bevorzugt zumindest 30 Sekunden, insbesondere bevorzugt 1 bis minuten durchgeführt. Die Äquilibrierung wird vorzugsweise mit einer Pufferlösung durchgeführt, der das zu reinigende flüssige Medium bzw. das zu reinigende Protein noch nicht zugesetzt ist.

Als weiterer Vorteil weist das im erfindungsgemäßen Verfahren verwendete Tonmaterial ein geringes Sedimentvolumen von weniger als 15 ml/2 g, vorzugsweise weniger als 10 ml/2 g, auf, d.h. es quillt in Wasser nur unwesentlich. Wegen seiner geringen Quellung ist bei der Zugabe des Tonmaterials zum zu reinigenden flüssigen Medium keine wesentliche Zunahme der Viskosität des die Proteine enthaltenden Mediums zu beobachten. Ferner lassen sich relativ große Mengen des Tonmaterials in das flüssige Medium einbringen, so dass auch aus Medien, welche eine hohe Konzentration der interessierenden Proteine aufweisen, eine Abtrennung dieser Proteine ohne weiteres möglich ist. Auch die Abtrennung des eingesetzten Tonmaterials von dem flüssigen Medium ist ohne weiteres durch Filtration möglich. Wegen des geringen Quellvermögens bewirkt das Tonmaterial keine Verstopfung des Filters. Dadurch ist beim Abfiltrieren des an Proteinen abgereicherten flüssigen Mediums nur ein vergleichsweise niedriger Druck erforderlich.

Als flüssiges Medium kann an sich jede Flüssigkeit verwendet werden, welche aus einer biologischen Quelle stammt bzw. Proteine enthält. Insbesondere eignet sich das Verfahren zur Aufarbeitung von Ansätzen aus Bioreaktoren, um die interessierenden biologisch wirksamen Stoffe aus großen Flüssigkeitsvolumina abzutrennen und aufzukonzentrieren. Eine andere Einsatzmöglichkeit ist beispielsweise die Abtrennung von Proteinen aus Körperflüssigkeiten, wie Harn, oder anderen biologischen Proben. Dies ermöglicht eine rasche Abtrennung und Reinigung von Proteinen beispielsweise aus Proben von Körperflüssigkeiten. Dazu wird die Probe, z.B. eine Körperflüssigkeit oder eine sonstige proteinhaltige Probe, auf das zuvor auf einen geeigneten pH-Wert äquilibrierte Tonmaterial gegeben. Die Proteine werden am Tonmaterial adsorbiert, während die flüssige Phase abgetrennt werden kann. Anschließend kann das Tonmaterial gespült werden, wozu bevorzugt eine Pufferlösung verwendet wird, deren pH-Wert in etwa dem pH-Wert entspricht, auf welchen das Proteine enthaltende flüssige Medium vor der Aufgabe auf das Tonmaterial eingestellt worden ist. Dadurch können Verunreinigungen ausgewaschen werden, während das Protein am Tonmaterial adsorbiert bleibt und auf diese Weise weiter gereinigt wird. Nach dem Spülschritt kann das Protein eluiert werden und liegt dann in einer angereicherten Form vor.

Gegebenenfalls müssen die Proben in üblicher Weise vorbereitet werden, indem beispielsweise eine Grobfiltration durchgeführt wird, oder die Probe gepuffert wird. Vorzugsweise wird die Probe auf einen pH-Wert gepuffert, der auch bei der Äquilibrierung des Tonmaterials eingestellt wurde. Derartige Probenvorbereitungen sind dem Fachmann bekannt. Das im erfindungsgemäßen Verfahren verwendete Tonmaterial wirkt vermutlich als Kationenaustauscher, wobei Alkali- oder Erdalkaliionen aus dem Tonmaterial freigesetzt werden und gegen Proteine ausgetauscht werden. Proteine umfassen geladene Aminosäurereste an der Proteinoberfläche, welche mit geladenen Gruppen an der Oberfläche des Tonmaterials wechselwirken können. Die Nettoladung eines Proteins ergibt sich aus dem pH-Wert der das Protein umgebenden Lösung, sowie dem pI-Wert des Proteins. Der pI-Wert eines Proteins entspricht dem pH-Wert, bei dem die Nettoladung des Proteins Null ist. Er ergibt sich aus der Aminosäurenkomposition sowie der Tertiärstruktur des Proteins. Im sauren pH-Bereich sind die Aminosäuren (bevorzugt die stärken basischen Aminosäuren Arginin, Lysin und Histidin) protoniert, und das Protein zeigt kationischen Charakter. Im basischen pH-Bereich tragen die sauren Gruppen Aspargin und Glutamin negative Ladungen, und das Protein liegt als Anion vor. Nahe am isoelektrischen Punkt ist die Wechselwirkung zwischen Protein und dem Tonmaterial gering und sie nimmt zu, je mehr sich der pH-Wert vom pI-Wert unterscheidet. Bevorzugt wird daher das Medium auf einen pH-Wert eingestellt, der sich um mindestens eine Einheit vom isoelektrischen Punkt des abzutrennenden Proteins unterscheidet. Weist das Protein beispielsweise einen pI-Wert von 5,5 auf, so wird die Isolierung des Proteins vorzugsweise bei pH-Werten unterhalb von etwa 4,5 durchgeführt.

Das im erfindungsgemäßen Verfahren verwendete Tonmaterial weist ein Porenvolumen von mehr als 0,35 ml/g, vorzugsweise mehr als 0,5 ml/g (bestimmt nach der BJH-Methode (kumulatives Porenvolumen für Poren mit einem Durchmesser im Bereich von 1,7 bis 300 nm)), eine spezifische Oberfläche (BET-Fläche) von mehr als 150 m²/g, vorzugsweise mehr als 180 m²/g, insbesondere bevorzugt mehr als 200 m²/g, und eine Ionenaustauschkapazität von mehr als 40 meq/100 g auf. Ferner zeichnet sich das Tonmaterial durch ein sehr geringes Quellvermögen aus. Das Quellvolumen entspricht vorzugsweise dem Sedimentvolumen. Geeignete analytische Methoden zur Bestimmung des Porenvolumens, der spezifischen Oberfläche, der Ionenaustauschkapazität sowie des Quellvolumens sind nachstehend bei den Beispielen angegeben.

Besonders bevorzugt werden Tonmaterialien verwendet, deren Ionenaustauschkapazität über 40 meq/100 g, vorzugsweise im Bereiche von 45 bis 75 meq/100 g liegt. Vorzugsweise weist das Tonmaterial eine spezifische Oberfläche (BET) im Bereich von 150 bis 280 m²/g, insbesondere bevorzugt im Bereich von 170 bis 260 m²/g auf. Das Porenvolumen des verwendeten Tonmaterials liegt vorzugsweise im Bereich von mehr als 0,35 ml/g, bevorzugt mehr als 0,5 ml/g, insbesondere bevorzugt im Bereich von 0,7 bis 1,1 ml/g, und ganz besonders bevorzugt im Bereich von 0,80 bis 1,0 ml/g.

Wie bereits weiter oben erläutert, quillt das im erfindungsgemäßen Verfahren verwendete Tonmaterial nur in sehr geringem Ausmaß, wodurch keine Probleme mit der Erhöhung der Viskosität einer wässrigen Suspension auftreten, wie dies beispielsweise bei Natriumbentonit im Allgemeinen beobachtet wird. Vorzugsweise beträgt das Sedimentvolumen, nachdem das Tonmaterial bei Raumtemperatur für drei Tage in Wasser stehen gelassen wurde, weniger als 15 ml/2 g, vorzugsweise weniger als 10 ml/2 g. Unter Raumtemperatur wird eine Temperatur von etwa 15 bis 25°C, insbesondere etwa 20°C, verstanden.

Als Tonmaterialien werden bevorzugt natürlich vorkommende naturaktive oder nicht-naturaktive Tonmaterialien verwendet, die vorzugsweise noch keiner chemischen Modifikation unterworfen wurden, insbesondere nicht mit starken Säuren dealuminiert wurden. Die Tonmaterialien können gegebenenfalls getrocknet und auf eine geeignete Korngröße gemahlen werden. Die Korngröße ergibt sich für den Fachmann aus der beabsichtigten Anwendung.

Neben den natürlich vorkommenden Tonmaterialien können an sich auch synthetisch hergestellte Tonmaterialien verwendet werden, welche die oben angegebenen Eigenschaften aufweisen. Solche Tonmaterialien können beispielsweise aus Wasserglas und einem geeigneten Schichtsilicat, wie Bentonit, hergestellt werden. Die Verwendung von Tonmaterialien, die aus natürlichen Quellen gewonnen wurden, ist jedoch bevorzugt.

Es werden Tonmaterialien verwendet, deren Aluminiumgehalt, bezogen auf das wasserfreie Tonmaterial, berechnet als Al₂O₃, weniger als 11 Gew.-% beträgt. Weiter beträgt der Aluminiumgehalt mehr als 4 Gew.-%, besonders bevorzugt mehr als 6 Gew.-%. Insbesondere bevorzugt liegt der Aluminiumgehalt im Bereich von 8 bis 10 Gew.-%. Tonmaterialien, die durch Extraktion mit starker Säure gewonnen werden, zeigen einen niedrigeren Al₂O₃-Gehalt als das im erfindungsgemäßen Verfahren verwendete Tonmaterial.

Besonders bevorzugt werden Tonmaterialien verwendet, welche nur eine geringe Kristallinität aufweisen, also an sich nicht der Klasse der Schichtsilicate zugeordnet werden. Die geringe Kristallinität lässt sich beispielsweise durch Röntgendiffraktometrie feststellen. Die besonders bevorzugten Tonmaterialien sind weitgehend amorph, d.h. sie zeigen im Röntgendiffraktogramm keine scharfen Peaks. Sie gehören daher nicht der Klasse der reinen Attapulgite oder Smektite an.

Ohne an diese Theorie gebunden sein zu wollen, nehmen die Erfinder an, dass das im erfindungsgemäßen Verfahren verwendete Tonmaterial ein Gerüst umfasst, das aus Kieselgel gebildet ist. In dieses relativ starre Gerüst ist ein Schichtsilicat eingelagert. Durch die Verankerung im Gerüst des Kieselgels kann das Schichtmineral stark quellen, ohne dass dadurch das Mineral in seiner Gesamtheit stark in seinem Volumen zunimmt. Das aufgequollene Schichtsilicat steht dann in hohem Ausmaß für eine Adsorption von Proteinen zur Verfügung. Das Kieselgel stellt die Hauptphase dar während die Tonphase den geringeren Anteil am Tonmaterial bildet. Das Tonmaterial ist vermutlich für das Kationenaustauschvermögen verantwortlich.

Das im erfindungsgemäßen Verfahren verwendete Tonmaterial weist in einer bevorzugten Ausführungsform eine besondere Porenradienverteilung auf. Der wesentliche Anteil des Porenvolumens des Tonmaterials wird dabei von Poren gebildet, welche einen Durchmesser von mindestens 14 nm aufweisen.

Besonders bevorzugt sind mindestens 40% des Gesamtporenvolumens (bestimmt gemäß der BJH-Methode, vergleiche unten) von Poren gebildet, die einen Durchmesser von mehr als 14 nm aufweisen. Bevorzugt werden mehr als 50%, und insbesondere bevorzugt mehr als 60% des Gesamtporenvolumens von Poren gebildet, die einen Durchmesser von mehr als 14 nm aufweisen. Die Porenradienverteilung bzw. das Gesamtporenvolumen wird durch Stickstoffporosimetrie (DIN 66131) und Auswertung der Adsorptionsisotherme nach der BJH-Methode (vergleiche unten) bestimmt.

Um eine Denaturierung des Proteins zu verhindern wird das Tonmaterial vor der Aufgabe des flüssigen Mediums auf einen pH-Wert von etwa 3,5 bis 9,0, insbesondere bevorzugt 4,0 bis 6,0 äquilibriert. Dazu wird das Tonmaterial in einem geeigneten Puffer, beispielsweise einem Citratpuffer, aufgeschlämmt oder beispielsweise eine aus dem Tonmaterial hergestellte Filterpackung oder Säule mit einem derartigen Puffer beaufschlagt. Der Puffer weist dabei vorzugsweise eine Konzentration im Bereich von 30 bis 100 mmol/l auf.

Gemäß einer weiteren Ausführungsform kann das Tonmaterial auch vor der Abtrennung von Proteinen aktiviert werden, indem es mit einer natriumhaltigen Verbindung, beispielsweise Soda, oder einer kaliumhaltigen Verbindung, z.B. Kaliumcarbonat, behandelt wird. Dadurch werden die zweiwertigen Kationen des Tonmaterials teilweise oder vorzugsweise ganz durch Natriumionen ausgetauscht. Für den Austausch der zweiwertigen Ionen gegen Natriumionen wird in der üblichen Weise vorgegangen, wie sie auch aus der Aktivierung von Calciumbentoniten bekannt ist. Das feuchte Tonmaterial, welches einen Feuchtegehalt von vorzugsweise zwischen 10 und 70 Gew.-%, vorzugsweise 45 bis 65 Gew.-% aufweist, wird mit einer Menge an Soda, die etwa 1,2 bis 2,5 Äquivalenten der Kationenaustauschkapazität des Tonmaterials entspricht, verknetet. Alternativ kann das Soda auch als Lösung auf das Tonmaterial aufgesprüht werden. Das Tonmaterial kann anschließend getrocknet und ggf. gemahlen werden. Durch die Aktivierung kann die Adsorptionskapazität des Tonmaterials für Proteine weiter gesteigert werden.

Die Behandlung des proteinhaltigen flüssigen Mediums kann im einfachsten Fall in.der Weise erfolgen, dass das Tonmaterial direkt oder in Form einer Suspension zu dem flüssigen Medium gegeben wird und nach einer Einwirkzeit, vorzugsweise im Bereich von 1 bis 30 Minuten, insbesondere bevorzugt 5 bis 20 Minuten, durch geeignete Verfahren, wie beispielsweise Filtration oder Zentrifugation, vom flüssigen Medium abgetrennt wird, welches dadurch an Proteinen abgereichert wird.

Gemäß einer weiteren Ausführungsform kann das flüssige Medium zur Abtrennung der Proteine durch eine Filterpackung geleitet werden, welche zumindest anteilig aus dem Tonmaterial gebildet ist. Die Filterpackung kann dabei an sich jede beliebige Form annehmen. Geeignet sind beispielsweise Filterpatronen, welche mit dem Tonmaterial gefüllt sind. Nachdem die Aufnahmekapazität der Filterpatrone erschöpft ist, kann diese gegen eine neue Patrone ersetzt werden. Die mit den Proteinen beladene Filterpatrone kann entweder verworfen werden oder bevorzugt einer Aufarbeitung zugeführt werden, in welcher die auf dem Tonmaterial adsorbierten Proteine wiedergewonnen werden.

Gemäß einer Ausführungsform wird die Filterpackung durch eine Anschwemmfiltration hergestellt. Das Tonmaterial wird dabei vorzugsweise mit einem Filterhilfsmittel, wie Kieselgur oder Perlit^{®}, gemischt. Ferner kann das Tonmaterial auch noch mit einem weiteren Adsorbens, wie Kieselgel, gemischt sein. Bei der Filtration bildet das Tonmaterial zusammen mit dem Filterhilfsmittel und gegebenenfalls weiteren Adsorbentien einen Filterkuchen aus, der das filtrationswirksame Medium für nachfolgende Trübstoffe und Filterhilfsmittelteilchen bildet. Diese Ausführungsform ist insbesondere dann geeignet, wenn Proteine als Störstoffe aus dem flüssigen Medium entfernt werden sollen.

Bevorzugt wird vor der direkten Filtration eine Filterhilfsmittelschicht als Primärschicht auf ein Filtermittel aufgebracht. Als Filtermittel kann beispielsweise ein Sieb mit einer geeignet geringen Maschenweite verwendet werden. Als Filterhilfsmittel zur Ausbildung einer Primärschicht kann beispielsweise Kieselgur verwendet werden. Dieser Prozess wird auch als Voranschwemmung bezeichnet. In der anschließenden Filtrationsphase wird der Suspension weiteres Filterhilfsmittel zugesetzt, das erfindungsgemäß das oben beschriebene Tonmaterial umfasst. Diese Filterhilfsmittel bilden gemeinsam mit den Trübstoffteilchen einen Filterkuchen aus, der als Sekundärschicht bezeichnet wird.

Gemäß einer weiteren Ausführungsform des erfindungsgemäßen Verfahrens wird dieses in Form einer Chromatographie durchgeführt. Dabei wird das Tonmaterial in eine Chromatographiesäule gepackt und das flüssige Medium, aus welchem die Proteine abgetrennt werden sollen, auf die Säule gegeben. Die Säule kann dann von einem Elutionsmittel durchströmt werden, wobei durch Adsorptionseffekte eine Auftrennung der Proteine in unterschiedliche Fraktionen möglich ist. Dies ist insbesondere interessant für die Abtrennung von Proteinen, welche in Bioreaktoren erzeugt wurden. Hier zeigen sich die Vorteile des im erfindungsgemäßen Verfahren verwendeten Tonmaterials, das sich durch ein sehr geringes Quellvermögen auszeichnet.

Um das Tonmaterial erfindungsgemäß in einer Säulenpackung einsetzen zu können, ist es zweckmäßig, dieses durch Absieben auf geeignete Teilchengrößen einzustellen. Besonders bevorzugt werden hierbei insbesondere die Feinanteile des Tonmaterials entfernt.

Bevorzugt wird das Tonmaterial auf eine Teilchengröße von > 10 µm, insbesondere > 20 µm, besonders bevorzugt > 30 µm, insbesondere in einem Bereich von 40 bis 300 µm eingestellt. Insbesondere bevorzugt weist das Tonmaterial auf einem Sieb der Maschenweite 45 µm einen Trockensiebrückstand von > 95 % der Einwaage, auf einem Sieb der Maschenweite 63 µm einen Trockensiebrückstand von > 80 % und auf einem Sieb der Maschenweite 150 µm einen Trockensiebrückstand von > 30 % auf.

Vorzugsweise dient das erfindungsgemäße Verfahren zur Gewinnung von Proteinen aus flüssigen Medien. Dazu wird das Protein, nachdem es auf dem Tonmaterial adsorbiert wurde und ggf. mit weiteren Spülschritten gereinigt wurde, wieder vom Tonmaterial eluiert. Die Eluierung der auf dem Tonmaterial adsorbierten Proteine kann nach verschiedenen Verfahren erfolgen. Beispielsweise kann das Protein durch eine Erhöhung der Salzkonzentration von den Bindungsstellen des Tonmaterials verdrängt werden. Weiter besteht die Möglichkeit, den pH-Wert der zur Elution verwendeten Lösung so einzustellen, dass sich die Ladung des adsorbierten Proteins ändert und es somit nicht mehr vom Tonmaterial gebunden wird. Wird für die Abtrennung des Proteins aus dem flüssigen Medium das Tonmaterial in Form eine Säule bereitgestellt, über die das flüssige Medium geleitet wird, kann zur Elution des adsorbierten Proteins beispielsweise ein Salzgradient über die Säule geleitet werden, so dass verschiedene Proteine bei unterschiedlicher Salzkonzentration eluiert werden, also eine Fraktionierung der adsorbierten Proteine erfolgt. Proteine werden unter anderem durch elektrische Wechselwirkungen und/oder van der Waals-Kräfte an das im erfindungsgemäßen Verfahren verwendete Tonmaterial gebunden. Bei der Eluierung mit einem Puffer mit hoher Salzkonzentration werden die elektrostatischen Wechselwirkungen zwischen dem Tonmaterial, welches als Kationenaustauscher wirkt, und dem Protein herabgesetzt. Das Protein kann daher vom Tonmaterial abgelöst und eluiert werden.

Bei einer weiteren Ausführungsform erfolgt die Eluierung der gebundenen Proteine durch eine Änderung des pH-Wertes. Dabei weist das Elutionsmittel einen zum flüssigen Medium unterschiedlichen pH-Wert auf.

Beispielsweise können Proteine bei einem pH aufgegeben werden, bei welchem sie in geladener Form vorliegen. Der pH-Wert der Lösung wird dabei vorzugsweise so eingestellt, dass er unterhalb des isoelektrischen Punktes des Proteins liegt, das Protein also eine positive Gesamtladung aufweist. Durch die Kationenaustauscherwirkung des Tonmaterials werden die Proteine gebunden. Das im erfindungsgemäßen Verfahren verwendete Tonmaterial wirkt also als Kationenaustauschermaterial. Wird nun der pH so weit angehoben, dass der isoelektrische Punkt des Proteins erreicht oder überschritten wird, weist das Protein in den meisten Fällen eine negative Gesamtladung auf. Das Protein wird daher wieder vom Tonmaterial abgelöst und kann eluiert werden.

Gegebenenfalls können dem Elutionsmittel Glycerin oder Polyglykole beigegeben sein. Diese Verbindungen weisen eine sehr hohe Affinität zum Tonmaterial auf und können daher die Ablösung des Proteins vom Tonmaterial erleichtern.

Das flüssige Medium umfasst bevorzugt Wasser als Lösungsmittel.

Das im erfindungsgemäßen Verfahren verwendete Tonmaterial kann alleine oder auch in Kombination mit einem weiteren Adsorptionsmaterial verwendet werden. Das weitere Adsorptionsmaterial ist vorzugsweise aus der Gruppe ausgewählt, die gebildet ist aus Kieselgel, Cellulose und Polyvinylpyrrolidon.

Das Tonmaterial und das weitere Adsorptionsmaterial liegen bevorzugt in einem Verhältnis zwischen 1:10 und 10:1 vor. In Abhängigkeit von der beabsichtigten Anwendung können jedoch auch Verhältnisse außerhalb des angegebenen Bereichs zur Anwendung gelangen.

Das im erfindungsgemäßen Verfahren verwendete Tonmaterial weist ein sehr hohes Tragevermögen für Wasser auf, wobei es dennoch fließfähig bleibt. Bevorzugt weist das Tonmaterial einen Wassergehalt von mehr als 30 Gew.-%, insbesondere mehr als 40 Gew.-%, insbesondere bevorzugt mehr als 50 Gew.-%, auf.

Das erfindungsgemäße Verfahren wird anhand der folgenden Beispiele sowie unter Bezugnahme auf die beigefügten Figuren näher erläutert. Dabei zeigt:
- Fig. 1:: eine Grafik, in welcher die pH-Abhängigkeit der Adsorption von Modellproteinen an das im erfindungsgemäßen Verfahren verwendete Tonmaterial gezeigt ist;
- Fig. 2:: Adsorptionsisothermen verschiedener Modellproteine an das im erfindungsgemäßen Verfahren verwendete Tonmaterial, aufgenommen in einem 50 mM Citrat-Puffer; und
- Fig. 3:: eine Grafik, in welcher die Beladung des im erfindungsgemäßen Verfahren verwendeten Tonmaterials in Abhängigkeit von der Auftragungsgeschwindigkeit für verschiedene Modellproteine dargestellt ist.

### Analysenmethoden

### Oberfläche/Porenvolumen:

Die Oberfläche der Tonmaterialien wurde an einem vollautomatischen Stickstoffporosimeter der Firma Micromeritics, Typ ASAP 2010, gemäß DIN 66131 durchgeführt. Das Porenvolumen wurde unter Anwendung der BJH-Methode ermittelt (I.P. Barrett, L.G. Joyner, P.P. Haienda, J. Am. Chem. Soc. 73 (1951), 373). Porenvolumina bestimmter Porengrößenbereiche werden durch Aufsummieren inkrementeller Porenvolumina bestimmt, die aus der Auswertung der Adsorptionsisotherme nach BJH erhalten werden. Das Gesamtporenvolumen nach der BJH-Methode bezieht sich auf Poren mit einem Durchmesser von 1,7 - 300 nm.

### Wassergehalt:

Der Wassergehalt der Produkte bei 105°C wurde unter Verwendung der Methode DIN/ISO 787/2 ermittelt.

### Elementaranalyse:

Diese Analyse beruht auf dem Totalaufschluss der Tonmaterialien bzw. des entsprechenden Produktes. Nach dem Auflösen der Feststoffe werden die Einzelkomponenten mit spezifischen Analysenmethoden, wie z.B. ICP, analysiert und quantifiziert.

### Ionenaustauschkapazität:

Zur Bestimmung der Kationenaustauschkapazität wird das zu untersuchende Tonmaterial über einen Zeitraum von 2 Stunden bei 105°C getrocknet. Danach wird das getrocknete Tonmaterial mit einem Überschuss an wässriger 2N NH₄Cl-Lösung eine Stunde unter Rückfluss zur Reaktion gebracht. Nach einer Standzeit von 16 Stunden bei Raumtemperatur wird filtriert, worauf der Filterkuchen gewaschen, getrocknet und vermahlen wird und der NH₄-Gehalt im Tonmaterial durch Stickstoffbestimmung (CHN-Analysator der Firma Leco) nach den Herstellerangaben ermittelt. Der Anteil und die Art der ausgetauschten Metallionen wird im Filtrat durch ICP-AES-Spektroskopie bestimmt.

### Röntgendiffraktometrie:

Die Röntgenaufnahmen wurden an einem hochauflösenden Pulverdiffraktometer der Firma Phillips (X'-Pert-MPD(PW 3040)) erstellt, das mit einer Cu-Anode ausgerüstet ist.

### Bestimmung des Sedimentvolumens:

Ein graduierter 100 ml-Messzylinder wird mit 100 ml destilliertem Wasser gefüllt. 2 g der zu vermessenden Substanz werden langsam und portionsweise, je etwa 0,1 bis 0,2 g, mit einem Spatel auf die Oberfläche des Wassers gegeben. Nach dem Absinken einer zugegebenen Portion wird die nächste Portion zugegeben. Nachdem die 2 g Substanz zugegeben und auf den Grund des Messzylinders abgesunken sind, wird der Zylinder für 1 Stunde bei Raumtemperatur stehen gelassen. Anschließend wird an der Graduierung des Messzylinders die Höhe des Sedimentvolumens in ml/2 g abgelesen. Für die Bestimmung des Sedimentvolumens nach dreitägiger Lagerung in Wasser wird der Probenansatz mit Parafilm^{®} verschlossen und für drei Tage bei Raumtemperatur erschütterungsfrei stehen gelassen. Danach wird an der Graduierung des Messzylinders das Sedimentvolumen abgelesen.

### Bestimmung des Trockensiebrückstandes:

Etwa 50 g des zu untersuchenden lufttrockenen Tonmaterials werden auf einem Sieb der Maschenweite 45 µm eingewogen. Das Sieb wird an einen Staubsauger angeschlossen, der über einen unter dem Siebboden kreisenden Saugschlitz alle Anteile, die feiner als das Sieb sind, durch das Sieb heraussaugt. Das Sieb wird mit einem Plastikdeckel abgedeckt und der Staubsauger eingeschaltet. Nach fünf Minuten wird der Staubsauger abgeschaltet und die Menge der auf dem Sieb verbliebenen gröberen Anteile durch Differenzwägung ermittelt.

### Bestimmung des Nasssiebrückstandes:

Es wird zunächst eine 5%-ige Suspension hergestellt, indem eine entsprechende Menge des zu untersuchenden Tonmaterials bei ca. 930 UpM ca. fünf Minuten in Wasser eingerührt wird. Die Suspension wird für weitere 15 Minuten bei ca. 1865 UpM gerührt und dann durch ein Sieb der gewünschten Maschenweite gegossen. Der Rückstand wird solange mit Leitungswasser gewaschen, bis das Waschwasser klar abläuft. Das Sieb mit dem Rückstand wird dann für fünf Minuten in ein Ultraschallbad gesetzt, um restliche Feinanteile zu entfernen. Der verbliebene Rückstand wird kurz mit Leitungswasser gewaschen und die Ultraschallbehandlung gegebenenfalls wiederholt, bis während der Ultraschallbehandlung keine Feinstoffe mehr in das Wasser übertreten. Das Sieb wird dann bis zur Gewichtskonstanz getrocknet. Zum Auswiegen wird der auf dem Sieb verbliebene Rückstand in eine gewogene Porzellanschale überführt.

### Charakterisierung der Tonmaterialien:

### a) Tonmaterial A:

Ein für das erfindungsgemäße Verfahren geeignetes Tonmaterial (Tonsil^{®} EX 1221 I; Süd-Chemie AG, Moosburg, DE, (Rohtonlager Res. No.: 03051)) wurde hinsichtlich seiner physikalisch-chemischen Eigenschaften untersucht. Die hierbei erzielten Ergebnisse sind in Tabelle 1 zusammengefasst.

**Tabelle 1: Physikalisch-chemische Analyse des Tonmaterials A**

| | | |
|---|---|---|
| Spezifische Oberfläche | (m²/g) | 219 |
| Porenvolumen¹ | (ml/g) | 0,881 |
| IUF | (meq/100g) | 52 |
| **Elementaranalyse:** | | |
| SiO₂ | (Gew.-%) | 70,6 |
| Fe₂O₃ | (Gew.-%) | 2,8 |
| Al₂O₃ | (Gew.-%) | 9, 8 |
| CaO | (Gew.-%) | 1, 4 |
| MgO | (Gew.-%) | 4, 1 |
| Na₂O | (Gew.-%) | 0,26 |
| K₂O | (Gew.-%) | 1,5 |
| TiO₂ | (Gew.-%) | 0,25 |
| Glühverlust (2h 1000°C) | | 7, 9 |
| **Summe** | **(Gew.-%)** | **98,6** |

| | | |
|---|---|---|
| ¹: kumulatives Porenvolumen nach BHJ für Poren mit einem Durchmesser zwischen 1,7 und 300 nm. | | |

Damit das erfindungsgemäße Silicat in einer Form vorliegt, in der sich gut Säulen packen lassen, wurde es auf Teilchengrößen > 45 µm abgesiebt . Das abgesiebte Material wies einen Trockensiebrückstand von > 95 % auf einem Sieb der Maschenweite 45 µm auf. Der Trockensiebrückstand auf 63 µm betrug 85 %, der auf 150 µm 30 %.

Weiter wurde das in Tabelle 1 charakterisierte Tonmaterial auf den Anteil am Porenvolumen untersucht, der von Poren mit bestimmten Radien gebildet wird. Die entsprechenden Daten sind in Tabelle 2 a bis c zusammengefasst.

**Tabelle 2a**

| Relative Anteile der Poren am Porenvolumen | | | | | |
|---|---|---|---|---|---|
| Bereich | 0 - 75 Å | 0 - 140 Å | 0 - 250 Å | 0 - 800 Å | > 800 Å |
| Anteil (%) | 10,3 | 19,3 | 34,1 | 78,0 | 22,0 |

**Tabelle 2b**

| Relative Anteile der Poren am Porenvolumen | | | | | |
|---|---|---|---|---|---|
| Bereich | 0 - 75 Å | 75 - 140 Å | 140 - 250 Å | 250 - 800 Å | > 800 Å |
| Anteil (%) | 10,3 | 9,0 | 14,8 | 43,9 | 22,0 |

**Tabelle 2c**

| Relative Anteile der Poren am Porenvolumen | | | | | | |
|---|---|---|---|---|---|---|
| Bereich | 0 - 75 Å | 75 - 800 Å | > 75 Å | > 140 Å | > 250 Å | > 800 Å |
| Anteil (%) | 10,3 | 67,7 | 89,7 | 80,7 | 65,9 | 22,0 |

### b) Tonmaterial B:

Die physikalisch-chemischen Eigenschaften eines weiteren Tonmaterials, das für die Durchführung des erfindungsgemäßen Verfahrens geeignet ist, sind in Tabelle 3 zusammengefasst.

**Tabelle 3**

| Physikalisch-chemische Analyse des Tonmaterials B | | |
|---|---|---|
| Spezifische Oberfläche | (m²/g) | 198 - 206 |
| Porenvolumen¹ | (ml/g) | 0,402 |
| IUF | (meq/100g) | 62 |
| **Elementaranalyse:** | | |
| SiO₂ | (Gew.-%) | 35,2 |
| Fe₂O₃ | (Gew.-%) | 0,93 |
| Al₂O₃ | (Gew.-%) | 4,6 |
| CaO | (Gew.-%) | 15,4 |
| MgO | (Gew.-%) | 16,0 |
| Na₂O | (Gew.-%) | 0,47 |
| K₂O | (Gew.-%) | 0,66 |
| TiO₂ | (Gew.-%) | 0,1 |
| Glühverlust (2h 1000°C) | | 25,4 |
| **Summe** | **(Gew. -%)** | **98,76** |

### c) Alkalische Aktivierung des Tonminerals A

Bei der Bestimmung der Gesamtionenaustauschkapazität (IUF) kann aus den Konzentrationen der Ionen im Eluat bestimmt werden, dass der Anteil von Na⁺ und K⁺ an der Gesamtkationenaustauschkapazität 32 % (Na⁺: 23 %, K⁺: 9 %) beträgt. Daraus lässt sich berechnen, dass man eine Menge von 2 % Soda benötigt, um eine stöchiometrische Aktivierung mit einwertigen Ionen zu erzielen.

Das in Tabelle 1 charakterisierte Tonmaterial A, wird zunächst in Stücke von weniger als 3 cm Durchmesser vorgebrochen. Der Wassergehalt wird ggf. durch Besprühen des gebrochenen Tonmaterials mit Wasser auf einen Wassergehalt von etwa 45 bis 65 Gew.-% eingestellt. 350 g des vorgebrochenen Tonmaterials A werden in eine Mischvorrichtung (z.B. einen Werner & Pfleiderer Mischer) gegeben und für eine Minute geknetet. Dann wird unter Weiterlaufen der Mischvorrichtung 2 Gew.-% festes Soda (bezogen auf wasserfreies Tonmaterial A) zugegeben und die Mischung für weitere 10 Minuten geknetet. Sofern die Mischung nicht ausreichend geschmeidig ist, kann destilliertes Wasser zugegeben werden, um eine ausreichend intensive Scherwirkung zu erhalten.

Die Knetmasse wird in kleine Stücke zerteilt und diese in einem Umlufttrockenschrank bei etwa 75 °C 2 bis 4 Stunden auf einen Wassergehalt von 10 ± 2 % getrocknet. Das Trockengut wird anschließend in einer Schlagrotormühle (z.B. einer Retsch-Mühle) über einem 0,12 mm Sieb vermahlen.

### Verwendete Modellproteine

Für die Untersuchungen zur Adsorption von Proteinen wurden die folgenden Modellproteine verwendet:

### α-Chymotrypsin (CHY):

α-Chymotrypsin ist ein Verdauungsenzym, welches aus der Bauchspeicheldrüse von Rindern gewonnen wird. Das Enzym besitzt eine Masse von 25,3 kDa. Der isoelektrische Punkt liegt zwischen pH 8,1 und 8,6.

### Humanes Serumalbumin (HSA) :

HSA ist ein globuläres Transportprotein, das als Carrier für Fettsäuren und Amphiphile aus dem Blut ins umgebende Gewebe dient. HSA ist aus drei Domänen aufgebaut, die ihrerseits aus 585 Aminosäuren mit einem Gesamtgewicht von 66,4 kDa bestehen. Der isoelektrische Punkt des Moleküls liegt bei pH 4,9.

### Alkalische Phosphatase (AP):

Alkalische Phosphatase ist eine unspezifische Phosphorsäuremonoesterase, die in allen Spezies von E. coli bis zum Menschen als Dimer identischer Subeinheiten vorkommt. AP ist ein Metalloprotein, das zwei Zn²⁺- und ein Mg²⁺-Ion enthält. Das in den Beispielen verwendete AP wurde aus der Darmschleimhaut von Kälbern isoliert. Der isoelektrische Punkt liegt bei pH 6,0. Das Molekülgewicht des Dimers beträgt 140 kDa.

### Beispiel 1: Adsorption von Proteinen im statischen System

### Äquilibrierung der Tonmaterialien

Jeweils 25,0 mg des oben charakterisierten Tonmaterials A werden in einem 50 ml-Reaktionsgefäß (Sarstedt AG & Co., Nümbrecht, DE) eingewogen. Es werden jeweils 10 ml der in Tabelle 4 angegebenen Probenvorbereitungspuffer zugegeben und das Reaktionsgefäß 30 Minuten im Ultraschallbad behandelt. Anschließend wird das Reaktionsgefäß mit der Suspension für eine Stunde auf einem Schütteltisch bei Raumtemperatur bei 100 UpM geschüttelt. Die Proben werden jeweils für 10 Minuten bei 4000 g zentrifugiert und anschließend der klare Überstand abpipettiert. Das Sediment wird in 10 ml bidestilliertem H₂O resuspendiert und 5 Minuten bei 100 UpM geschüttelt. Die Suspension wird dann für 10 Minuten bei 4.000 g zentrifugiert und der klare Überstand abpipettiert. Das äquilibrierte Tonmaterial wird anschließend für 16 Stunden bei 60°C getrocknet.

**Tabelle 4: verwendete 100 mM Puffer**

| | |
|---|---|
| Puffer pH 3, 0 | 100 mM Natriumphosphatpuffer |
| Puffer pH 4,0 | 100 mM Natriumcitratpuffer |
| Puffer pH 5,0 | 100 mM Natriumacetatpuffer |
| Puffer pH 6,0 | 100 mM MES-Puffer |
| Puffer pH 7,0 | 100 mM Trispuffer |
| Puffer pH 8,0 | 100 mM Trispuffer |
| Puffer pH 9,0 | 100 mM Trispuffer |

### Vorbereitung der Modellproteine

Von den Modellproteinen werden jeweils Stammlösungen mit einer Konzentration von 2 mg / ml in bidest. H₂O angesetzt. Die Proteinlösungen werden im Verhältnis 1:1 mit den in Tabelle 4 angegebenen 100 mM Puffern der pH-Werte 3 bis 8 zu einem Gesamtvolumen von 20 ml verdünnt.

### Erstellung von Kalibrationskurven zur UV-photometrischen Bestimmung der Proteinkonzentrationen

Die photometrische Quantifizierung von Proteinen basiert auf der Messung der UV-Absorption der aromatischen Aminosäuren Tyrosin (Phenolgruppe, 275 nm), Tryptophan (Indolgruppe, 279 nm) und in geringerem Maße Phenylalanin (257 nm) bei 280 nm. Es können je nach der Aminosäurezusammensetzung Konzentrationen zwischen 20 und 3000 µg·ml Protein nachgewiesen werden.

Um eine unbekannte Proteinkonzentration zu ermitteln, wird zunächst die Absorption für eine Kalibrationsreihe von Lösungen bekannter Proteinkonzentration bei 280 nm bestimmt. Aus dem linearen Zusammenhang zwischen Absorption und Proteinkonzentration lässt sich die unbekannte Proteinkonzentration errechnen.

Für die photometrische quantitative Auswertung werden von den Modellproteinen Standardreihen in den in Tabelle 5 angegebenen 50 mM Puffern der entsprechenden pH-Werte hergestellt. Die Standards werden 3 Stunden bei 4°C und 100 UpM auf einem Schütteltisch inkubiert. Nach beendeter Inkubation werden die Proben für 10 Minuten bei 4000 g zentrifugiert und die Extinktion des klaren Überstands bestimmt. Aus den ermittelten Werten wird eine Eichkurve erstellt.

**Tabelle 5: verwendete 50 mM Puffer**

| | |
|---|---|
| Puffer pH 3,0 | 50 mM Natriumphosphatpuffer |
| Puffer pH 4,0 | 50 mM Natriumcitratpuffer |
| Puffer pH 5,0 | 50 mM Natriumacetatpuffer |
| Puffer pH 6,0 | 50 mM MES-Puffer |
| Puffer pH 7,0 | 50 mM Trispuffer |
| Puffer pH 8,0 | 50 mM Trispuffer |
| Puffer pH 9,0 | 50 mM Trispuffer |

### a) Untersuchung der pH-Abhängigkeit der Proteinadsorption

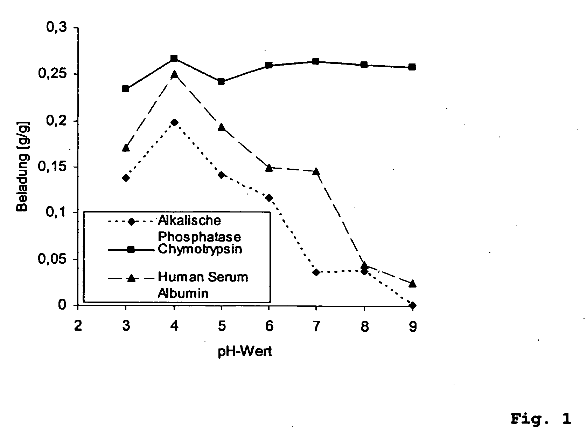

Zu jeweils 25 mg des äquilibrierten Tonmaterials werden jeweils 20 ml der wie oben beschrieben vorbereiteten Lösung des Modellproteins gegeben. Die Proben werden 3 Stunden bei 4 °C und 100 UpM auf einem Schütteltisch inkubiert. Nach beendeter Inkubation werden die Proben für 10 Minuten bei 4000 g zentrifugiert. Die Konzentration der Modellproteine in den Probenüberständen wird photometrisch bei 280 nm gegen die Standardreihe des Proteins im jeweiligen Puffer bestimmt. Die Beladung der Tonmaterialien errechnet sich aus der Differenz der Proteinkonzentration im Überstand vor und nach der Inkubation, sowie der Masse des Tonmaterials und dem Volumen der Proteinlösung. Die Ergebnisse der Messungen sind als Mittelwerte einer Doppelbestimmung in Figur 1 gegen die pH-Werte aufgetragen. Die Beladung des Tonmaterials A ist für alle Modellproteine bei pH 4 in Citratpuffer am höchsten.

### b) Aufnahme von Proteinabsorptionsthermen

Die Aufnahme von Adsorptionsisothermen wird in Abhängigkeit der Gleichgewichtsbeladung des Tonmaterials A von der Modellproteinkonzentration im Überstand untersucht.

Die Versuche werden für die jeweiligen Modellproteine bei einem pH-Wert von 4 in einem 50 mM Citrat-Puffer durchgeführt. Dies entspricht den Bedingungen, bei welchen bei der Untersuchung der pH-Abhängigkeit der Proteinadsorption der maximale Wert ermittelt worden war.

Für die Aufnahme der Adsorptionsisothermen wird zunächst eine Stammlösung des betreffenden Modellproteins mit einem Proteingehalt von 3 mg/ml in 50 mM Citrat-Puffer hergestellt. Die Proteinstammlösung wird dann gemäß der in Tabelle 6 angegebenen Vorschrift mit 50 mM Citrat-Puffer verdünnt.

**Tabelle 6: Vorschrift zur Verdünnung von Proteinstammlösungen für die Aufnahme von Protein-Adsorptionsisothermen**

| **Versuchs-Nr.** | **Volumen Proteinstammlsg. [ml]** | **Volumen Puffer [ml]** | **c(Protein) [mg·ml⁻¹]** | **Massenverhältnis Protein/Adsorber** |
|---|---|---|---|---|
| 1 | 2 | 33 | 0,17 | 0,24 |
| 2 | 5 | 30 | 0,43 | 0,60 |
| 3 | 8 | 27 | 0, 69 | 0,96 |
| 4 | 12 | 23 | 1,03 | 1,44 |
| 5 | 16 | 19 | 1,37 | 1,92 |
| 6 | 20 | 15 | 1, 71 | 2,40 |
| 7 | 24 | 11 | 2, 06 | 2,88 |

Die verdünnten Lösungen der Modellproteine werden dann zu jeweils 25 mg Tonmaterial A gegeben, das wie oben beschrieben mit 50 mM Citrat-Puffer äquilibriert worden war.

Für die Bestimmung der Konzentration der Modellproteine wird für jedes Modellprotein eine Standardreihe in einem 50 mM Citrat-Puffer angesetzt. Die Proben und die Standardlösung werden für 3 Stunden bei 4°C und 100 UpM auf einem Schütteltisch inkubiert und anschließend für 10 Minuten bei 4.000 g zentrifugiert.

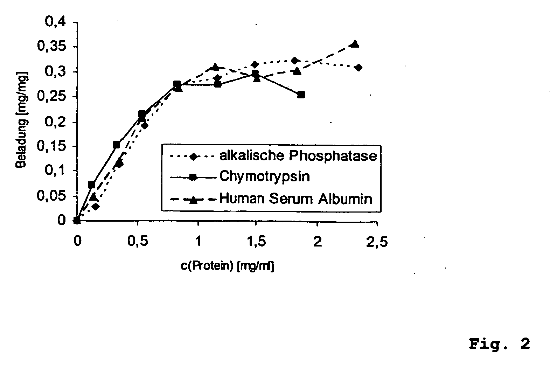

Für die Standardreihe der Proteine wird die Extinktion bestimmt und daraus eine Eichkurve erstellt. Die Beladung der Tonmaterialien wird durch photometrische Quantifizierung der Proteine im Überstand der zentrifugierten Proben ermittelt. Die Messwerte sind als Mittelwerte einer Doppelbestimmung in Figur 2 dargestellt.

### Beispiel 2: Adsorption von Proteinen im dynamischen System

### Äquilibrierung der Tonmaterialien

In einem 1 ml-Eppendorfhütchen werden jeweils 100 mg Tonmaterial A mit jeweils 1 ml der in Tabelle 5 angegebenen 50 mM Puffer suspendiert und die Suspension mit einer Pipette in eine mit einem Stempel nach unten abgeschlossene Chromatographiesäule (15 x 50 mm mit 10 µm PTFE-Stopfen) pipettiert. Das zweite Säulenendstück wird aufgesetzt und die Säule mit einer FPLC-Anlage so verbunden, dass sie von der mobilen Phase von unten nach oben durchströmt wird. Die FPLC-Pumpe wird auf die Flussrate eingeregelt, für welche die Kapazität des Adsorbers ermittelt werden soll. Der bewegliche Stempel der Säule wird während der Äquilibrierung mit 20 ml 50 mM Puffer langsam handfest angezogen und anschließend um eine viertel Gewindedrehung entlastet.

### Bestimmung der Proteinbindungskapazität

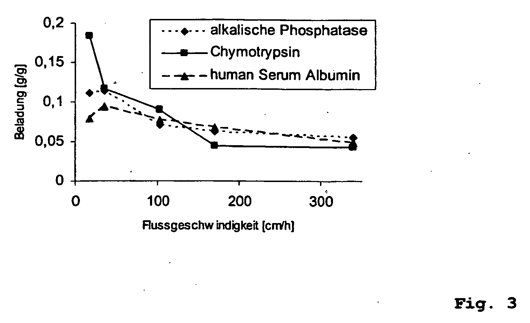

25 ml einer Lösung des jeweiligen Proteins in 50 mM Citrat-Puffer werden bei variablen Flussraten durch eine mit 100 mg Tonmaterial A gepackte Säule gepumpt. Der Proteingehalt im Durchlauf wird photometrisch bei 280 nm gegen eine Standardreihe des Modellproteins in einem 50 mM Citrat-Puffer ermittelt. Die für das Tonmaterial A ermittelten flussratenabhängigen Beladungen mit den Modellproteinen sind in Figur 3 als Mittelwerte einer Doppelbestimmung dargestellt.

### Beispiel 3 Elution von humanem Serumalbumin

Zur Elution von an Tonmaterial A gebundenem HSA werden 2 verschiedene Puffer verwendet. Alle Chromatographieschritte werden bei einer Flussrate von 1 ml/min durchgeführt.
Elutionspuffer 1: 50 mM Citrat, 1 M NaCl in bidest. H₂O, pH 4
Elutionspuffer 2: 50 mM Na₂HPO₄ in bidest. H₂O, pH 7,2

100 mg des Tonmaterials A werden im Durchflussbetrieb mit 25 ml einer HSA-Lösung beladen, welche eine Konzentration von 1 mg/ml in 50 mM Citratpuffer pH 4 aufweist. Nach der Beladung wird die Säule mit 20 ml 50 mM Citratpuffer pH 4 gespült. Für die Elution des HSA werden 35 ml Elutionspuffer durch die Säule geleitet. Der Proteingehalt des Durchlaufs, der Wasch- und der Elutionsfraktion wird photometrisch bei 280 nm bestimmt. Die Messergebnisse sind für beide Elutionspuffer als Mittelwerte einer Doppelbestimmung in Tabelle 7 wiedergegeben.

**Tabelle 7: Proteingehalte in Durchlauf, Wasch- und Elutionsfraktion bei der Elution von HSA vom Tonmaterial A mit 2 verschiedenen Elutionspuffern**

| **Puffer** | **HSA-Gehalt [mg]** | | | **Elution [% Beladung]** |
|---|---|---|---|---|
| | **Durchlauf** | **Waschfraktion** | **Elutionsfraktion** | |
| Elutionspuffer 1 | 15,5 | 1, 7 | 2, 1 | 31 |
| Elutionspuffer 2 | 15,4 | 1, 7 | 7,0 | 89 |

Mit dem Elutionspuffer 2 konnte im Vergleich zum Elutionspuffer 1 die dreifache Menge Protein eluiert werden.

## Patentansprüche

1. Verfahren zur Abtrennung von Proteinen aus flüssigen Medien, wobei
- ein Proteine enthaltendes flüssiges Medium bereitgestellt
- ein Tonmaterial bereitgestellt wird, welches:
- eine spezifische Oberfläche von mehr als 150 m²/g,
- ein Porenvolumen von mehr als 0,35 ml/g,
- einen Ionenaustauschkapazität von mehr als 40 meq/100 g,
- ein Sedimentvolumen in Wasser von weniger als 15 ml/2 g, und
- bezogen auf das wasserfreie Tonmaterial (atro),einen Al₂O₃-Gehalt im Bereich von 4 bis 11 Gew.-% aufweist;
- das Tonmaterial auf einen pH-Wert von 3,5 bis 9,0 äquilibriert wird,
- das flüssige Medium mit dem äquilibrierten Tonmaterial behandelt wird, und
- das gereinigte, an Proteinen abgereicherte flüssige Medium von dem Tonmaterial abgetrennt wird.

2. Verfahren nach Anspruch 1, wobei das Tonmaterial, bezogen auf das wasserfreie Tonmaterial (atro), einen Al₂O₃-Gehalt von mehr als 6 Gew.-% aufweist.

3. Verfahren nach Anspruch 1 oder 2, wobei das Tonmaterial, bezogen auf das wasserfreie Tonmaterial (atro), einen SiO₂-Gehalt von mehr als 65 Gew.-% aufweist.

4. Verfahren nach einem der vorhergehenden Ansprüche, wobei mindestens 40 % des Porenvolumens des Tonmaterials von Poren bereitgestellt werden, die einen Porendurchmesser von mindestens 14 nm aufweisen.

5. Verfahren nach einem der vorhergehenden Ansprüche, wobei das Sedimentvolumen des Tonmaterials nach Stehenlassen bei Raumtemperatur für 3 Tage in Wasser weniger als 15 ml/2g, vorzugsweise weniger als 10 ml/2 g, beträgt.

6. Verfahren nach einem der vorhergehenden Ansprüche, wobei das Tonmaterial keiner Oberflächenaktivierung mit Säure unterworfen worden ist.

7. Verfahren nach einem der vorhergehenden Ansprüche, wobei das Tonmaterial mit einem Puffer äquilibriert wird.

8. Verfahren nach Anspruch 7, wobei der Puffer eine Konzentration im Bereich von 30 bis 100 mmol aufweist.

9. Verfahren nach einem der vorhergehenden Ansprüche, wobei das Tonmaterial durch Behandlung mit einer alkalimetallhaltigen Verbindung, insbesondere natriumhaltigen Verbindung, aktiviert worden ist.

10. Verfahren nach einem der vorhergehenden Ansprüche, wobei das flüssige Medium zur Abtrennung der Proteine durch eine Filterpackung geleitet wird, welche zumindest anteilig aus dem Tonmaterial gebildet ist.

11. Verfahren nach einem der vorhergehenden Ansprüche, wobei das flüssige Medium zur Abtrennung der Proteine über eine Chromatographiesäule geleitet wird, deren Packung im Wesentlichen aus dem Tonmaterial gebildet ist.

12. Verfahren nach einem der vorhergehenden Ansprüche, wobei nach der Abtrennung des gereinigten, an Proteinen abgereicherten flüssigen Mediums die an das Tonmaterial gebundenen Proteine mit einem Elutionsmittel eluiert werden.

13. Verfahren nach Anspruch 12, wobei das Elutionsmittel einen zum flüssigen Medium unterschiedlichen pH-Wert aufweist.

14. Verfahren nach einem der vorhergehenden Ansprüche, wobei das Tonmaterial eine Korngröße von > 45 µm aufweist.

15. Verfahren nach einem der vorhergehenden Ansprüche, wobei das Tonmaterial mit einem weiteren Adsorptionsmaterial verwendet wird.

16. Verfahren nach Anspruch 15, wobei das weitere Adsorptionsmaterial ausgewählt ist aus der Gruppe, die gebildet ist aus Kieselgel, Cellulose und Polyvinylpyrrolidon.

17. Verfahren nach Anspruch 15 oder 16, wobei das Tonmaterial und das weitere Adsorptionsmaterial bezogen auf das Gewicht in einem Verhältnis zwischen 1:10 und 10:1 vorliegen.

## Claims

1. Method for separating proteins from liquid media, wherein
- a protein-containing liquid medium is provided,
- a clay material is provided, which has:
- a specific surface area of more than 150 m²/g,
- a pore volume of more than 0.35 ml/g,
- an ion exchange capacity of more than 40 meq/100 g,
- a sediment volume in water of less than 15 ml/2 g, and
- relative to the anhydrous clay material (absolutely dry), an Al₂O₃ content in the range of from 4 to 11 wt.-%;
- the clay material is equilibrated to a pH of from 3.5 to 9.0,
- the liquid medium is treated with the equilibrated clay material, and
- the purified, protein-depleted liquid medium is separated from the clay material.

2. Method according to claim 1, wherein, relative to the anhydrous clay material (absolutely dry), the clay material has an Al₂O₃ content of more than 6 wt.-%.

3. Method according to claim 1 or 2, wherein, relative to the anhydrous clay material (absolutely dry), the clay material has an SiO₂ content of more than 65 wt.-%.

4. Method according to one of the previous claims, wherein at least 40 % of the pore volume of the clay material is provided by pores which have a pore diameter of at least 14 nm.

5. Method according to one of the previous claims, wherein the sediment volume of the clay material, after being left to stand at room temperature for 3 days in water, is less than 15 ml/2 g, preferably less than 10 ml/2 g.

6. Method according to one of the previous claims, wherein the clay material has not been subjected to surface activation with acid.

7. Method according to one of the previous claims, wherein the clay material is equilibrated with a buffer.

8. Method according to claim 7, wherein the buffer has a concentration in the range of from 30 to 100 mmol.

9. Method according to one of the previous claims, wherein the clay material has been activated by treatment with an alkali-metal-containing compound, in particular sodium-containing compound.

10. Method according to one of the previous claims, wherein the liquid medium is passed, for the separation of the proteins, through a filter pack at least a proportion of which is formed by the clay material.

11. Method according to one of the previous claims, wherein the liquid medium is passed, for the separation of the proteins, over a chromatography column the packing of which is formed substantially from the clay material.

12. Method according to one of the previous claims, wherein after the separation of the purified, protein-depleted liquid medium, the proteins bound to the clay material are eluted with an eluent.

13. Method according to claim 12, wherein the eluent has a different pH from the liquid medium.

14. Method according to one of the previous claims, wherein the clay material has a particle size of > 45 µm.

15. Method according to one of the previous claims, wherein the clay material is used with a further adsorption material.

16. Method according to claim 15, wherein the further adsorption material is selected from the group which is formed by silica gel, cellulose and polyvinylpyrrolidone.

17. Method according to claim 15 or 16, wherein, relative to the weight, the clay material and the further adsorption material are present in a ratio of between 1:10 and 10:1.

## Revendications

1. Procédé de séparation de protéines d'un milieu liquide,
- un milieu liquide contenant des protéines étant mis à disposition,
- une matière argileuse étant mise à disposition, laquelle présente :
- une surface spécifique supérieure à 150 m²/g,
- un volume poreux supérieur à 0,35ml/g,
- une capacité d'échange ionique supérieure à 40 meq/100 g,
- un volume de sédiments dans l'eau inférieur à 15 ml/2 g, et
- par rapport à la matière argileuse exempte d'eau (atro), une teneur en Al₂O₃ de l'ordre de 4 à 11% en poids ;
- la matière argileuse étant équilibrée à une valeur pH de 3,5 à 9,0,
- le milieu fluide étant traité avec la matière argileuse équilibrée et
- le milieu liquide purifié, appauvri en protéines étant séparé de la matière argileuse.

2. Procédé selon la revendication 1, en rapport à la matière argileuse exempte d'eau (atro), la matière argileuse présentant une teneur en Al₂O₃ supérieure à 6 % en poids.

3. Procédé selon l'une quelconque des revendications 1 ou 2, en rapport à la matière argileuse exempte d'eau (atro), la matière argileuse présentant une teneur en Si₂O₃ supérieure à 65 % en poids.

4. Procédé selon l'une quelconque des revendications précédentes, au moins 40 % du volume poreux de la matière argileuse étant mis à disposition par des pores présentant un diamètre de pores d'au moins 14 nm.

5. Procédé selon l'une quelconque des revendications précédentes, après repos à température ambiante pendant 3 jours dans de l'eau, le volume de sédiments de la matière argileuse étant inférieur à 15 ml/2g, de préférence inférieur à 10 ml/2 g.

6. Procédé selon l'une quelconque des revendications précédentes, la matière argileuse n'étant soumise à aucune activation superficielle à l'acide.

7. Procédé selon l'une quelconque des revendications précédentes, la matière argileuse étant équilibrée avec un tampon.

8. Procédé selon la revendication 7, le tampon présentant une concentration de l'ordre de 30 à 100 mmol.

9. Procédé selon l'une quelconque des revendications précédentes, la matière argileuse ayant été activée par un composé contenant des métaux alcalins, notamment un composé contenant du sodium.

10. Procédé selon l'une quelconque des revendications précédentes, pour la séparation des protéines, le milieu liquide étant dirigé à travers un bloc de filtres qui est au moins formé proportionnellement dans la matière argileuse.

11. Procédé selon l'une quelconque des revendications précédentes, pour la séparation des protéines, le milieu liquide étant dirigé à travers une colonne chromatographique, dont le bloc est formé essentiellement dans la matière argileuse.

12. Procédé selon l'une quelconque des revendications précédentes, après la séparation du milieu liquide purifié, appauvri en protéines, les protéines liées à la matière argileuse étant éluées avec un produit d'élution.

13. Procédé selon la revendication 12, le produit d'élution présentant une valeur pH différente de celle du milieu liquide.

14. Procédé selon l'une quelconque des revendications précédentes, la matière argileuse présentant une grosseur de grain > 45 µm.

15. Procédé selon l'une quelconque des revendications précédentes, la matière argileuse étant utilisée avec une autre matière d'adsorption.

16. Procédé selon la revendication 15, l'autre matière d'adsorption étant choisie dans le groupe qui est formé par le gel de silice, la cellulose et la polyvinylpyrrolidone.

17. Procédé selon la revendication 15 ou 16, la matière argileuse et l'autre matière d'adsorption se présentant dans une proportion comprise entre 1:10 et 10:1 en rapport au poids.
